# EUROPEAN PATENT SPECIFICATION

(11) **EP 2 831 003 B1**
(45) Date of publication and mention of the grant of the patent: **28.10.2020**
(21) Application number: 13720015.0
(22) Date of filing: 28.03.2013
(51) Int. Cl.: C02F 3/28, C12M 1/107

(54) **APPARATUS FOR THE PRODUCTION OF BIOGAS AND RELATED METHOD**
VORRICHTUNG ZUR HERSTELLUNG VON BIOGAS UND ENTSPRECHENDES VERFAHREN
APPAREIL POUR LA PRODUCTION DE BIOGAZ ET PROCÉDÉ S'Y RAPPORTANT

(30) Priority: 29.03.2012 IT MI20120516
(43) Date of publication of application: 04.02.2015
(73) Proprietor: Sereco Biotest di Bilenka Oksana S.A.S., 06121 Perugia (IT)
(72) Inventor: POLETTI, Antonio, I-06121 Perugia (PG) (IT); POLETTI, Roberto, I-06121 Perugia (PG) (IT); POLETTI, Luca, I-06121 Perugia (PG) (IT)
(74) Representative: Ercolani, Simone Pietro
(86) International application number: PCT/IB2013/000551
(87) International publication number: WO 2013/144703

(56) References cited:
- EP-A2- 0 213 691
- WO-A1-2008/099227
- DE-A1- 19 624 268
- DE-A1-102010 010 294

## Description

### FIELD OF THE INVENTION

The present invention relates to an apparatus for the production of biogas and related method.

In particular, the invention relates to an apparatus for producing biogas by an anaerobic reactor with separated chambers.

### KNOWN PREVIOUS ART

Various devices for the anaerobic digestion are known and reported in literature such as, for example, the system called as CSTR (Continuous Stirred Tank Reactor), or the UASB, (Upflow anaerobic sludge blanket) system (Morgan-Sagastume et al. 1997) or the TIS (Tank-in-series) system, described in documents US2011 / 0200954 and IT1380337. In particular, the TIS system comprises a plurality of small receptacles hydraulically communicating one to each other, without the realization of upper and lower openings for the passage of the biomasses through such small receptacles.

"Anaerobic Baffled Reactors" (ABR) are also know, which comprise a series of chambers separated one from each other by suitable consecutive vertical deflectors (also known with the English term of "baffles"), provided with upper and lower passages such to allow the passage of the influent, that is to say the organic material or biomass introduced into the reactor, from above and beneath the deflectors themselves.

The paper "The use of the anaerobic baffled reactor (ABR) for wastewater treatment: a review", published in 1999 in Water Research, Vol. 33, No. 7, pp 1559-1578, ELSEVIER, teaches that within each of the ABR reaction chambers, as the organic material passes from a chamber to another, several processes occur mediated by the presence of bacteria in charge to carry out activity of transforming the organic material itself. The organic material crosses four transformation steps: the hydrolysis step, in which the organic molecules undergo cleavage in simpler compounds such as, monosaccharides and amino acids; the acidogenesis step, in which the further cleavage in even simpler molecules occurs, such as the volatile fatty acids (for example the acetic, propionic, butyric and valeric acid), with production of ammonia, carbon dioxide and hydrogen sulphide as by-products; the acetogenesis step, during which simple molecules produced in the previous stage are further digested producing carbon dioxide, hydrogen and mainly acetic acid; and, at last, the methanogenesis step, with the production of methane, carbon dioxide and water.

DE19624268A discloses a process and assembly which converts especially organic refuse into gas for energy conversion. Liquid or flowing bulk solids carrying an organic load passes under control from the inlet to the outlet, through a horizontal fermentation assembly which is subdivided into a number of chambers. All of the chambers have an open aperture to a common gas chamber, such that the fermentation unit forms a common gas chamber for the gas. Each chamber is separated from its neighbour by a wall and incorporates assemblies such as heaters, mixers, valves and push-blades. The first chamber receives fresh air enabling it to operate as an anaerobic hydrolysis reactor. One or all chambers are supplied with a nutrient. Contaminated wastes are introduced via an inlet to a mixer.

In the known ABR reactors methane is one of the various products of the treatment and is produced in too low amounts for an economic exploitation thereof.

In addition, ABR reactors are not free from drawbacks.

The most recurring drawbacks of which is known from literature about the use of ABR reactors are the low rising velocity of liquids and gases, which implies the need of building not too deep reactors, a poor homogenization of the influent within the various compartments, frequent uncontrolled proliferations of the bacterial flora, in regions of the reactor with abrupt reduction or mutation of the bacterial flora in other regions of the reactor. This significantly reduces the overall efficiency of the apparatus for producing biogases and in some instances causes an excessive foam formation, which can lead to the biogas production stoppage inside the reactor or significant troubles about hydraulics.

Object of the present invention is to realize an apparatus and method able to solve the above described known art problems and which can lead to an effective, reliable biogas production and to such an extent that it can be commercially exploited.

Again, object of the present invention, is to realize an apparatus for producing biogas which is structurally simple and able to be realized also from the known ABR anaerobic reactors.

Further object of the invention is to realize an apparatus for producing biogas by anaerobic digestion of organic material, which can be employed with the most different types of organic material or mixtures thereof.

These and other objects are reached by the invention, which relates to an apparatus for producing biogas by the anaerobic digestion of organic material, comprising at least one anaerobic reactor provided with a loading section for said organic material and a plurality of reaction chambers, arranged in line and connected one to each other by lower and upper alternate passages, for the outflow of said organic material through each of said chambers of said plurality of reaction chambers, and means to convey the biogas produced within said one or more reaction chambers outside of said at least one reactor, characterized in that said at least one reactor comprises, as well, means for connecting one or more reaction chambers one to each other for the recirculation of said organic material between the mentioned one or more reaction chambers.

Note that said alternate passages are, alternatively, also of the upper and lower type, according to the typology of materials to be introduced.

With respect to the known art reactors the present invention is characterized by the absence of a solid sedimentation final compartment, since the solids recirculation, by the afore mentioned recirculation means, directly occurs between the chambers delimited by the deflectors. Such recirculation means allow the blending and the recirculation of the sludge from the downstream compartments towards the upstream compartments - with respect to the direction of the influent flow - in order to improve the results in the various biological stages of the anaerobic digestion process and increase the biogas productivity, reducing the sludge production since they are consumed more effectively and with higher yield.

In fact it has been surprisingly observed that a recirculation conceived, on the other hand, as an outlet of the liquid from the final stage only, in the system rear, can cause the definitive loss of solids in the final compartment and an excessive increase of the mixing in the starting compartment, which thing can determine a substantial disruption of the bacterial micro communities designated for the execution of the different methanogenic conversion of incoming biomass.

Definitely, in presence of uncontrolled blending, it is possible to come back to a condition of single stage digestion with loss of the separation between acidogenics and methanogenics, with significant reduction of the reactor overall efficacy. The proposed invention solves such problems, since the blend obtained in the reactor by the afore mentioned recirculation means occurs in a controlled way such to compensate for the problems of the known art reactors and obtaining, at the same time, a great biogas production and a consistent energetic saving because of the absence of moving mechanical members.

Therefore such a solution allows, by the use of said recirculation means, to transfer organic material not yet fully digested from a chamber to another of the reactor and, therefore, to submit it again to the anaerobic digestion processes carried out in chambers separated from the pickup chambers. This allows to obtain an optimal control over the steps of the anaerobic digestion process occurring within each chamber of the reactor. In this way, the bacterial load present in each reaction chamber can be monitored and any problem which can lead to a system stop, or to a reaction efficacy reduction, can be avoided, due to the fact, for example, that the bacterial flora increases in excess in a chamber or decreases in excess in another reaction chamber.

In particular, it has to be noted that the recirculation and redistribution direction of the organic material between said one or more chambers of said plurality of reaction chambers is opposite to the direction the organic material covers within said reactor, when it forcedly outflows through each one of said chambers of said plurality of reaction chambers. In particular, said recirculation means are preferably placed outside of the reactor.

According to the invention, said recirculation means - as mentioned - placed outside of said at least one reactor, comprise at least one main line fluidically connecting one or more reaction chambers one to each other, at least one or more inflow/ outflow lines for said organic material connected, at a first end, to said at least one main line and, at least a second end, to at least one chamber of said one or more reaction chambers, at least one recirculation pump for said organic material operating within said at least one main line and/ or said one or more inflow/ outflow lines, and valve means for connecting by control said one or more reaction chambers to said at least one main line of fluidic connection. In particular, said at least one second end of said one or more inflow / outflow lines is arranged beneath, that is to say on the bottom of, said one or more chambers of said plurality of chambers, for the pickup of the more solid component of said organic material from the reaction chamber, that is to say the component which needs to be reintroduced in another reaction chamber since not yet fully digested, or to make the organic material inflow inside the reaction chamber from the bottom of said reaction chamber . In a further embodiment, there is a further second end, in addition to the one placed on the bottom, however arranged at the level of the head of said organic material present in said one or more chambers, for the pickup of the liquid component of said organic material, or the foam, or the superficial crust of said organic material. In particular, in this embodiment means are present for breaking the crust forming on the surface of the organic material under digestion, due to the cooling caused by the produced gas and which crosses the organic material. Such means for breaking the crust are placed on the ceiling or one or more side walls of one or more of the reaction chambers of the reactor. In practice, said breaking means comprise either a blading rotatable around a vertical axis, if such means are arranged on the ceiling of said one or more chambers, or a blading rotatable around a horizontal axis, in case such means are arranged on one or more side walls of one or more of the reaction chambers of the reactor. The activation of such crust breaking means is controlled by sensors or, alternatively, by an operator. In accordance with a further embodiment, said crust breaking means comprise at least one Venturi effect crushing device, placed within at least one reaction chamber of said one or more reaction chambers. In particular, such crushing device comprises at least one first duct at least partially submerged in said organic material, and at least one second duct having an outlet section for additional organic material under pressure; said outlet section is arranged within said at least one first duct, submerged in said at least one organic material contained in said at least one reaction chamber. Said at least one second duct, as said above, carries organic material under pressure deriving from either the same chamber wherein said crushing device is positioned or from the outside thereof, such that such an organic material comes out from said outlet section and, due to the depression created in said first duct, drags along the crust formed on the surface of the organic material. Such crust breaking means, exploiting the Venturi effect, cause the blend of possible particles floating towards the bottom and bring, at the same time, to the breaking of the superficial crust.

According to the invention, said apparatus comprises at least one pre-treatment tank of said organic material and at least one line for the controlled supply through said at least one loading section of said at least one reactor with said organic material deriving from said at least one pre-treatment tank. In this way, the organic material is loaded within the reactor upon request according to production needs, thus constituting an effective tank for said reactor.

According to a particular embodiment of the invention, said at least one pre-treatment tank is provided with stirring means for said organic material loaded therein. In practice, such an organic material can remain in said tank for several hours, even beyond twenty four hours, during which it is subjected to at least the hydrolysis process, thus facilitating the following biological and chemical processes then occurring within the reactor. Advantageously the stirring within the reactor is able to promote the beginning of such biological and chemical processes since it continuously blends the organic material and also makes a partial grinding and maceration of the organic material itself.

According to a further embodiment of the invention, said at least one supply line comprises at least one pump and at least one opening/ closing valve to allow/ prevent the passage of said organic material from said at least one pre-treatment tank to said at least one reactor. Furthermore, said supply line comprises, as well, at least one auxiliary branch line for reintroducing the organic material into said pre-treatment tank. In this way, besides the material stirring a blending of the organic material contained within the said at least one pre-treatment tank can be made as well.

Specifically said pump present along said supply line is of the grinding type, able, therefore, to homogenize and crumble vegetal biomasses with high density and coarse and uneven grade grain size.

According to the invention, said means of controlled recirculation comprise at least one first connecting line for transferring at least part of said organic material in said at least one main line, to said at least one pretreatment tank. This becomes extremely advantageous since in this way not only the processes within each reactor chamber, but also what happens within the pre-treatment tank, can be controlled.

In practice, said pre-treatment tank, which is apart from the reactor, has the function to start the maceration and hydrolysis processes of the biomass intended for the anaerobic digestion. The dilution of the biomass is partially, or completely, carried out with the reactor waste water, in order to recover both heat and active bacterial flora. The recirculation rate of the waste water in the pre-treatment tank for the dilution is set by a suitable controlling system, in order to avoid the build-up of biotoxic by-products, such as for example ammonium, which are continuously monitored.

Furthermore, said means of controlled recirculation comprise, as well, at least one second connection line for transferring at least part of the organic material contained in one or more of said reaction chambers, or in said at least one main line, to the lagoon for the disposal of the exhausted material, or other destination, such as, for example, the final storage.

Again, said recirculation means comprise, as well, one or more evacuation lines of the organic material, or foams, or other like material, when a certain level within one or more chambers of said plurality of chambers is reached. In practice, each of said one or more evacuation lines connect at least one reaction chamber to said at least one main line.

According to the invention, the recirculation means comprise at least one unit for controlling the flow rates of part of the organic material recirculated between said one or more reaction chambers, or transferred from said one or more reaction chambers, or said at least one main line, to said at least one pre-treatment tank, or said lagoon, as a function of the information coming from one or more sensors of temperature and / or pH or flow rate of the biogas or of density of organic material or the like. Such sensors allow to effectively control the flow of organic material transferred between one chamber and another or between the reactor and the pre-treatment tank, or between the reactor and the lagoon, or the storage. This allows not only to improve the reactor operation, but also the operation of the pre-treatment tank and thus to increase the apparatus efficiency and the biogas production extent. Said controlling unit, as afore said, provides means for measuring the biomass density which comprise an impeller provided with vanes, of which the energy consumption is monitored. An increase or decrease of the energy consumption of the impeller, at constant velocity of the latter, allows to highly easily determine, although indirectly, an increase or decrease of the density of the biomass present in the reactor. Note that such means for measuring the density can be employed also independently from the apparatus for producing biogas object of the present invention.

Said at least one reactor further comprises means for heating one or more of the reaction chambers in a controlled way. In practice, in order to aid the digestion processes of the biomass introduced within the reactor, the chambers are conveniently brought to suitably controlled temperatures.

In particular, the heating means of the chambers comprise floor heating members of the type comprising one or more pipes in which a heating liquid flows.

According to an alternative embodiment such heating means are outside of the apparatus reactor.

Again, in accordance with a further embodiment of the invention, said heating means comprise heating members implemented within said one or more deflectors separating said reaction chambers one from each other. Preferably, such heating members are realized in the deflectors provided with upper openings. In particular, each deflector is empty inside and is crossed by said heating liquid. Furthermore, each deflector is partitioned by one or more partitions to realize a serpentine path within the deflector itself. Furthermore such heating means comprise at least one feed line of the heating fluid reaching said at least one wall and at least one return line for said heating fluid coming out from said at least one deflector. Such a solution allows to uniformly radiate the heat within one or more chambers of the reactor chambers. Furthermore, differently from the floor heating means, wherein the maintenance of the heating members has to be done periodically due to the fact that the latter are coated with hardened organic material which significantly reduces the radiated thermal power, the heating members realized directly in the deflectors do not need maintenance and allow to develop a remarkable thermal power that is uniformly distributed along the whole height of the reaction chamber on which they act.

It has to be noted that, in a particular embodiment, said reactor is provided with the above mentioned heating means, but free from the afore mentioned recirculation means for said organic material. In this case, therefore, an embodiment is obtained wherein said apparatus for producing biogas by the anaerobic digestion of organic material, comprises at least one anaerobic reactor provided with a loading section for said organic material and a plurality of reaction chambers arranged in line and connected one to each other by deflectors provided with lower and upper alternate passages, for the forced outflow of said organic material through each of said chambers of said plurality of reaction chambers, and means to convey the biogas produced within said one or more reaction chambers outside of said at least one reactor; said apparatus is characterized in that said at least one reactor comprises said heating means for said one or more chambers. In such an embodiment the means for the controlled recirculation of said organic material between one or more chambers of said plurality of reaction chambers are not present or are present dependently to the presence of the afore mentioned heating means for said at least one reactor.

Again, always according to a further embodiment of the invention, said reactor further comprises means for changing the volume of one or more chambers of said at least one reactor and/ or the volume of said at least one reactor. In this way the amount of biomass which can be loaded inside one or more chambers of said reactor and/ or said at least one reactor can be increased or decreased, also as a function of the type of organic material to employ for the biogas production or of the request of energy production.

According to an embodiment of the invention, said one or more deflectors of said reaction chambers are shiftably constrained to said reactor such to change the volume of said one or more reaction chambers.

In a further embodiment of the invention, said one or more deflectors are rotatably constrained to said at least one reactor such to change the volume of said one or more reaction chambers. In particular, the deflector rotation around a pin, for example, has two effects. Firstly, it changes the volume of the two chambers which are separated by said deflector and, secondly, the geometry of the chambers and thus the path the biomass followed within the reactor with the consequence of also modifying the load losses the biomass is subjected to during the reactor crossing. In fact, once the deflector is rotated, the flow of organic material within that specific chamber can be slowed or accelerated, thus increasing or decreasing the biomass passage time in that specific biological compartment or - more generally - in the reactor.

In addition, according to a specific embodiment of the invention, said means for changing the volume of said reactor comprise a separable wall constrained to the reactor itself. Such a wall, preferably the one arranged in the position opposite to the position wherein the loading section for said reactor is comprised, can be separated by the reactor for allowing the connection to the reactor of additional reaction chambers so to widen the volume of the reactor itself and increase the power available by the apparatus itself.

Note that, in a particular embodiment, said at least one reactor is provided with said means for changing the volume of said one or more reaction chambers and/ or said at least one reactor, but it is free from the said recirculation means for said organic material. Therefore in this case an embodiment is obtained wherein said apparatus for producing biogas by the anaerobic digestion of organic material, comprises at least one anaerobic reactor provided with a loading section for said organic material and a plurality of reaction chambers arranged in line and connected one to each other by deflectors provided with lower and upper alternate passages, for the forced outflow of said organic material through each one of said chambers of said plurality of reaction chambers, and means to convey the biogas produced within said one or more reaction chambers outside of said at least one reactor; said apparatus is characterized in that said at least one reactor comprises means for changing the volume of said one or more chambers and/ or said at least one reactor. In such an embodiment the means for the controlled recirculation of said organic material between one or more chambers of said plurality of reaction chambers are not present, or they are present dependently to the presence of the afore mentioned means for changing the volume.

Finally, said at least one reactor and/ or said at least one pre-treatment tank are realized and sized such to be transportable; preferably, the reactor and/ or pre-treatment tank sizes are compatible with sizes of the containers which can be transported by sea or land.

According to the invention also a method is provided for producing biogas by a production apparatus according to one or more of the claims 1 to 13, comprising the step of: a) loading said at least one reactor with said organic material; b) carrying out the crossing of said plurality of reaction chambers by said organic material; characterized in that, together with said step b), the method comprises, as well, the step of c) recirculating said organic material contained in said at least one reactor between said one or more chambers of said plurality of reaction chambers.

Furthermore, before said step a) it is comprised the step of d) filling said at least one pre-treatment tank with said organic material and the step of e) supplying in a controlled way by at least one supply line said at least one reactor with said organic material coming from said at least one pre-treatment tank. Again, said method comprises the further step of f) transferring at least part of the organic material contained in said at least one main line, to said at least one pre-treatment tank, or said lagoon or the storage.

Finally, said step c) and/ or f) comprises the further step of g) adjusting the flow rate of said organic material recirculated between said one or more reaction chambers of said plurality of reaction chambers, or transferred from said one or more reaction chambers, or said at least one main line, to said at least one pre-treatment tank, or said lagoon, as a function of the information coming from one or more sensors of biogas temperature and/ or pH or flow rate or of density of contained organic material.

Again, said step b) comprises the further step of g) heating in a controlled way said one or more chambers of said plurality of reaction chambers.

Finally, the method comprises the further step of h) changing the volume of said one or more chambers of said plurality of reaction chambers and/ or changing the volume of said at least one reactor.

In a particular embodiment, said method for producing biogas by a production apparatus according to one or more of the claims from 1 to 13, comprises the step of: a) loading said at least one reactor with said organic material; b) forcing the crossing of said plurality of reaction chambers by said organic material; characterized by comprising the further step of h) changing the volume of said one or more chambers of said plurality of reaction chambers and/ or said at least one reactor.

### BRIEF DESCRIPTION OF THE DRAWINGS

These and other aspects of the present invention will be more evident to the field technician from the following description of a preferred embodiment of the present invention, provided by way of example and not of limitation, referring to the attached figures, in which:
figure 1 is a schematic view of an ABR reactor according to known art;
figure 2 is a schematic view of the hydraulic circuit of the apparatus for producing biogas according to a first embodiment of the invention;
figure 3 is a schematic view of the hydraulic circuit of the apparatus for producing biogas according to a second embodiment of the invention;
figure 4 is a perspective view of the apparatus for producing biogas of figure 3;

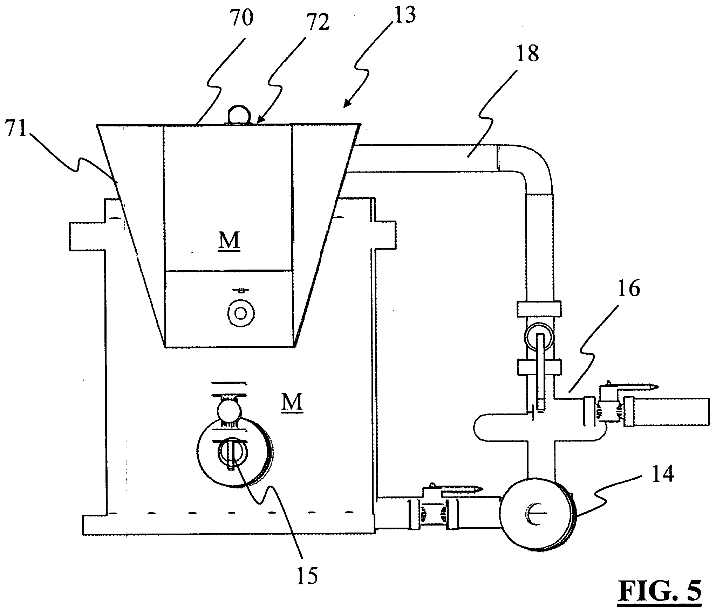

figure 5 is a front view of the pre-treatment tank according to the invention;
figure 6 is a side view of a wall of the reactor wherein the presence of a heating member is provided;
figure 7a is a side view of a shiftable deflector according to the invention;
figure 7b is a side view of a rotatable deflector according to the invention;

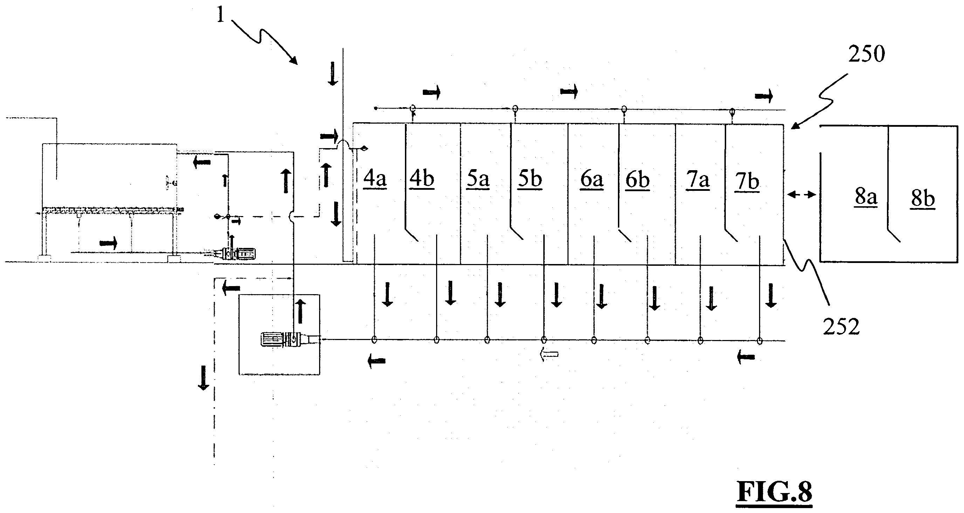

figure 8 is a schematic view of a reactor provided with means for changing the reactor volume;
figure 9 is a schematic view of a particular embodiment of the means for breaking the surface crust.

### DETAILED DESCRIPTION OF SOME EMBODIMENTS OF THE PRESENT INVENTION

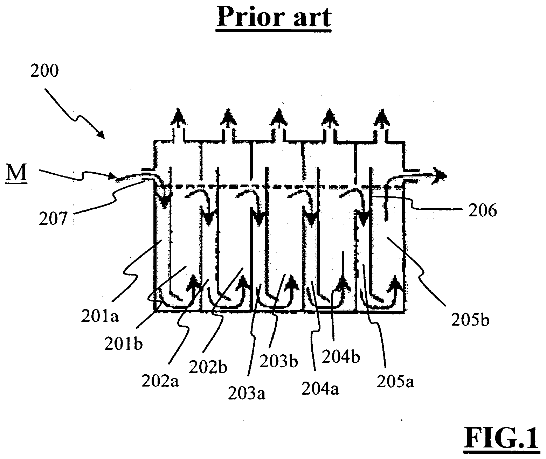

In figure 1 a known anaerobic reactor of the ABR type is shown. Such a reactor 200, as above mentioned, is constituted of reaction chambers 201a, 201b, 202a, 202b, 203a, 203b, 204a, 204b, 205a and 205b arranged in series and separated one from each other, as mentioned above, by deflectors 205 provided with upper and lower passages. Note that, according to known art, two consecutive reaction chambers, delimited by two upper passages, or also said spillway passages, constitute a biological compartment wherein mainly one step of the biological process of transforming the biomass introduced in the reactor itself occurs. The chambers 201a and 201b are the first biological compartment whereas the chambers 205a and 205b constitute the last biological compartment. The number of biological compartment can vary according to the type of biomass to be subjected to anaerobic digestion and according to the fluid dynamic features to be imparted to the biomass itself. Lower and upper passages, independently from their sequence, impart to the effluent a substantially sinusoidal path, that is to say a flow which is directed from bottom to top and vice versa. Such a reactor is, as well, provided with a supply section 207 through which the continuous load of the biomass occurs, and with a dedicated gas line adapted to collect and convey the biogas produced upon biological and chemical processes, which develop within the reactor, towards suitable energy exploitation systems. The organic material subjected to digestion can be zootechnical slurry (bovine, of swine, chicken droppings), agro-food industry wastes, dedicated energy crops, organic domestic wastes or a mixture thereof.

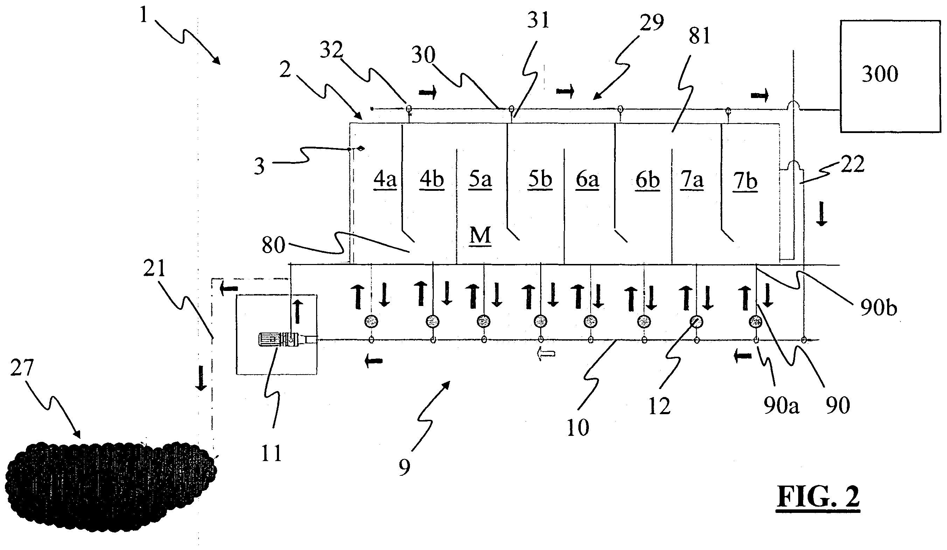

Referring generally to the attached figures, the apparatus 1 for producing biogas by the anaerobic digestion of organic material M, according to a first embodiment of the invention shown in figure 2, comprises an anaerobic reactor 2 provided with a loading section 3 for said organic material M and eight reaction chambers 4a, 4b, 5a, 5b, 6a, 6b, 7a, 7b arranged in line and connected one to another by deflectors 79 provided with lower 80 and upper 81, alternate passages, to allow the forced outflow of said organic material M from the loading section 3 until reaching the last chamber 7b, opposite to the chamber 4a wherein said loading section 3 is present, by line crossing each one of said chambers 4b, 5a, 5b, 6a, 6b, 7a. Note that each reaction chamber 4a, 4b, 5a, 5b, 6a, 6b, 7a, 7b is partitioned from the adjacent one by deflectors 79 provided with lower 80 and upper 81 alternate passages, in particular four lower passages 80 and three upper passages 81, or spillway passages, such to make the organic material M outflow into the reactor 2 through a substantially sinusoidal, or serpentine, path. Such a solution belongs, none the less, to the known art and is common to the above described ABR type reactors generically shown in figure 1. Note that, always according to known art, the deflector 79 arranged in the reactor 2 such to accommodate said lower passage 80 has a terminal member sloped with respect to the vertical wall, for example obtuse angled greater than 90°, such that it can take a substantially L-shape. Such a shape is advantageously employed for improving the outflow of the biomass M through the lower passage 80 between two adjacent reaction chambers.

Furthermore, said reactor 2 is provided with means 29 for convey the biogas produced within said reaction chambers 4a, 4b, 5a, 5b, 6a, 6b, 7a, 7b outside of said at least one reactor. Such conveying means 29 comprise a duct 30 placed over the ceiling of the reactor 2 and a suction fan (not shown) suctioning said biogas picking it up from the anaerobic reactor 2. Furthermore, such conveying means 29 comprise at least four lines 31 for extracting the biogas from four different chambers 4b, 5b, 6b, 7b of the reactor 2. Each extraction line 31 is provided with suitable shutters 32 whose closing/ opening can be controlled based on the production requirements. For example, in case wherein the produced biogas was excessively rich in carbon dioxide, the two shutters 32 corresponding to the extraction lines connected to chambers 4b and 5b could be closed and the further two shutters connected to chambers 6b and 7b could be open. Note that, in any case, the number of said extraction lines as well as that of the shutters can also differ from the number provided in the herein described embodiment without thereby departing from the protection scope of the present invention.

Advantageously, said anaerobic reactor 2 comprises, as well, means 9 for the controlled recirculation of the organic material M contained in said reactor 2 between each one of the reaction chambers 4a, 4b, 5a, 5b, 6a, 6b, 7a, 7b composing the reactor. Note that, despite herein and in the following a reactor 2 will be described, whose recirculation means 9 allow to recirculate the organic material through all reaction chambers 4a, 4b, 5a, 5b, 6a, 6b, 7a, 7b of which the reactor 2 is composed, however an embodiment wherein the recirculation occurs, for example, only between two or three chambers of the reactor 2 still falls within the protection scope of said patent. Furthermore, it has to be mentioned that a reactor 2 comprising a number different from eight reaction chambers mentioned in this embodiment still falls within the protection scope of the present invention.

According to the herein described embodiment, said recirculation means 9 comprise, outside of said reactor 2, a main line 10, which fluidically connects said eight reaction chambers 4a, 4b, 5a, 5b, 6a, 6b, 7a, 7b one to each other, one inflow/ outflow line 90 for each reaction chamber 4a, 4b, 5a, 5b, 6a, 6b, 7a, 7b and, further, a recirculation pump 11 for said organic material M, placed inside said main line 10, specifically upstream of the chamber 4a which presents said loading section 3 for said organic material M. Note that each outflow/ inflow line 90 is connected, at one end 90a, to said main line 10 and, at the other end 90b, to a reaction chamber 4a, 4b, 5a, 5b, 6a, 6b, 7a, 7b of said plurality of reaction chambers composing said reactor 2.

Furthermore, said recirculation means 9 also comprise valve means 12 to connect by control said reaction chambers 4a, 4b, 5a, 5b, 6a, 6b, 7a, 7b, to said fluidic connecting main line 10. Said valve means 12, which in this case are solenoid valves, are arranged on said outflow/ inflow lines 90 and operate such to allow, or not, the flow of organic material through said outflow/ inflow lines 90. In practice, according to the herein described embodiment, by acting such to open, or close, the solenoid valves 12 upon specific control by the operator, or pre-set control, and by suitably activating the afore mentioned recirculation pump 11, the recirculation of the organic material M can be obtained with a flow running from the chamber placed furthest from the recirculation pump 11, for example the chamber 6b, to a chamber placed closer to the recirculation pump 11, for example the reaction chamber 5b. This is due to the specific positioning of the recirculation pump 11 with respect to the main line 10 and the reaction chambers 4a, 4b, 5a, 5b, 6a, 6b, 7a, 7b of the reactor 2. In practice, due to the above described configuration represented in the hydraulic layout of figure 2, once the solenoid valves 12 are opened, thus allowing the passage of the organic material along the respective inflow/ outflow line 90 along which they are placed, the organic material will always displace from one reaction chamber to another in such a direction that the flow is directed from the furthest chamber from the pump to the chamber nearest to the pump (see in this regard the arrows at the main line 10). In general terms, the direction of the flow rate of the organic material M recirculated through the reaction chambers is opposed to the feed direction of the organic material M within the reactor 2. Such a feed direction of the organic material, in the herein illustrated embodiment, goes from the chamber 4a to the chamber 7b, thus the recirculation direction goes from the chamber 7b to the chamber 4a.

Of course different hydraulic layout, wherein the flow of organic material recirculated through said one or more reaction chambers can follow both directions, exploiting, for example, several recirculation pumps or other herein not described hydraulic layouts, fall within the protection scope of the present invention as well.

In the herein described embodiment, said outflow/ inflow lines 90 are arranged such that the end 90b connected to said chambers 4a, 4b, 5a, 5b, 6a, 6b, 7a, 7b is placed on the bottom of said chambers 4a, 4b, 5a, 5b, 6a, 6b, 7a, 7b. In this way, each time the controlled recirculation of the organic material between one chamber and another is operated, in the outflow chamber the more solid component of said organic material is picked up, that is the component needing to be reintroduced in another reaction chamber since not yet fully digested during the anaerobic process. In a herein not shown embodiment, the ends 90b of the inflow/ outflow lines 90 are also arranged over said reaction chambers 4a, 4b, 5a, 5b, 6a, 6b, 7a, 7b at a height corresponding to the maximum level of head the organic material is intended to reach, when the latter present in the reactor 2, for the pickup of the more liquid component of said organic material, or the foam or crust which could form on the surface of the organic material. In particular, in this embodiment means 600 are present for breaking the crust 606 forming on the surface of the organic material under digestion due to the cooling down caused by the passage of the gas produced during the anaerobic digestion. Such means for breaking the crust are placed on the ceiling of the reaction chamber 4a of the reactor 2. Such breaking means comprise a blading (not shown) with vertical axis which is moved in a controlled way.

Alternatively, such means 600 for breaking the crust are placed on the wall of the reaction chamber 4a of the reactor 2. Such means 600 for breaking the crust comprise a blading (not shown) with horizontal axis which is moved in a controlled way.

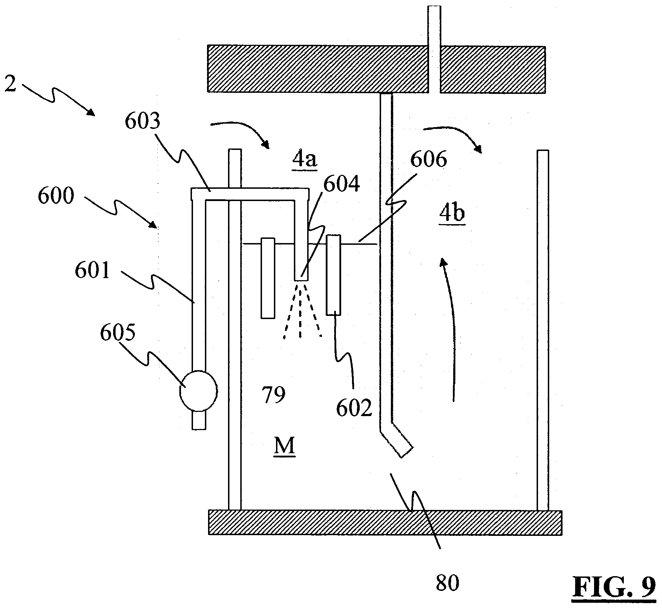

In accordance to a further embodiment shown in figure 9, said means 600 for breaking the crust 606 comprise a Venturi effect crushing device 601, placed within the reaction chamber 4a of said reactor 2. In particular, such crushing device 601 comprises a first duct 602, partially submerged in said organic material M, and a second curved duct 603 having an outlet section 604 for additional organic material under pressure. Such outlet section 604 is placed within said first duct 602, submerged in said organic material M contained in said reaction chamber 4a. Said second duct 603, as mentioned above, carries some organic material M under pressure, due for example to the use of the pump 605, coming from the outside of the reaction chamber 4a, so that such an organic material M comes out from said outlet section 604 and, thanks to the depression created in said first duct 602, drags along the crust formed on the surface of the organic material M. In an alternative embodiment, such an organic material M introduced under pressure through said inlet section 604, is picked up from the same reaction chamber 4a.

It has to be said that, although the herein described embodiments comprising means for breaking the crust are only present in the first chamber 4a, however an embodiment describing a reactor 2 comprising means for breaking the crust in several reaction chambers 4a, 4b, 5a, 5b, 6a, 6b, 7a, 7b, at the limit in all the reaction chambers, still falls within the protection scope of the present invention.

Note that, despite in the herein described embodiment the inflow/ outflow lines 90 have been designed for all the chambers 4a, 4b, 5a, 5b, 6a, 6b, 7a, 7b of the reactor 2, however an embodiment wherein such inflow/ outflow lines 90 were designed for some chambers only would still fall within the protection scope of the present invention.

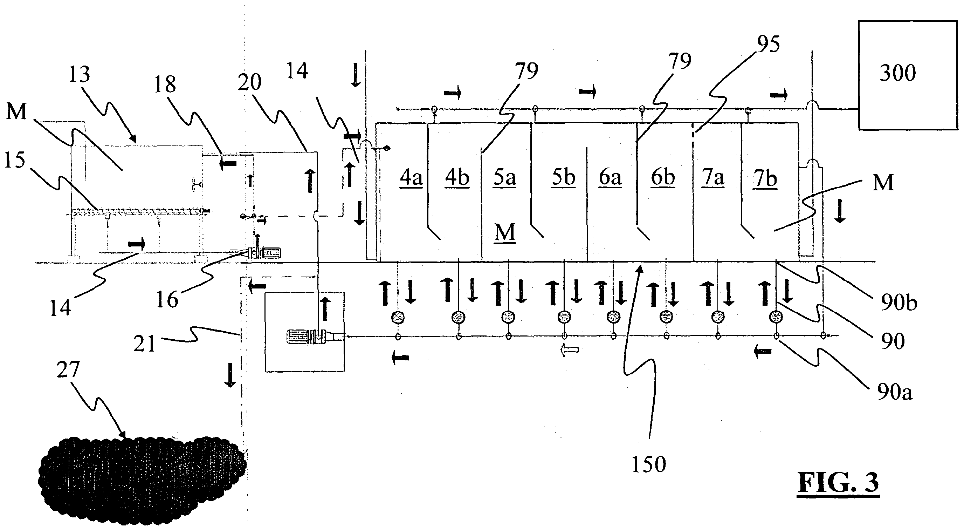

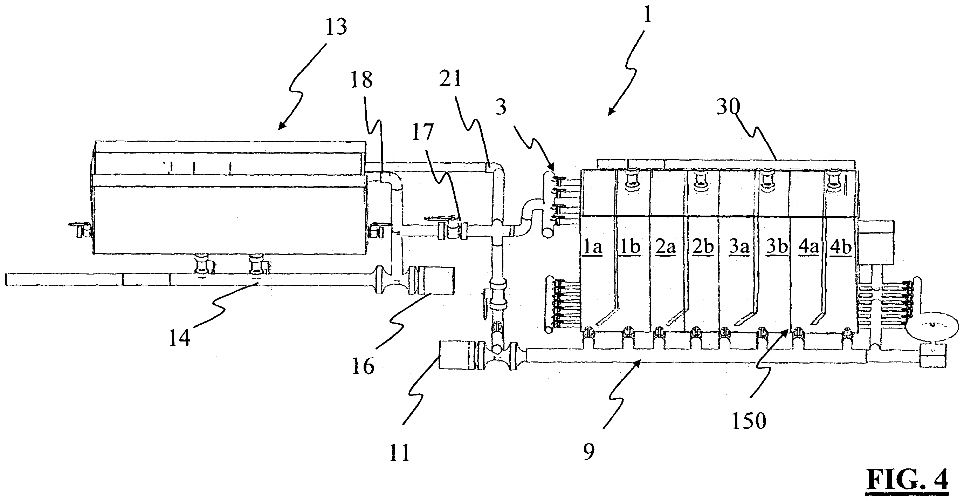

Again, according to a further embodiment of the invention shown in figures 3 and 4, said apparatus 1 comprises a pre-treatment tank 13 of said organic material M and a supply line 14 for the controlled supply through said loading section 3 of said reactor 2 with said organic material M and coming from said pre-treatment tank 13. Thereby this allows to obtain a continuous or, anyway, controlled flow of organic material M from said pre-treatment tank 13 to said reactor 2.

The pre-treatment tank 13 comprises a loading section 70 and a hopper 71 which are adapted to aid the incoming of the organic material M into the tank 13. Such a loading section, each time the organic material has been loaded within the pre-treatment tank 13, is closed by suitable covering means 72.

In particular, said pre-treatment tank 13 is provided with stirring means 15 for said organic material M loaded therein. For example, in the case shown in figure 3, such stirring means 15 comprise a mixing auger conveyor or, alternatively, wall stirrers. In this way the organic material M does not hold keep still within the pre-treatment tank 13 before being transferred to the reactor 2, but it is continuously stirred so that somehow the biological and chemical processes of cleavage of organic material are promoted in such a way that the organic material, already suitably prearranged to undergo the processes of anaerobic digestion then occurring in the reactor 2, reaches the reactor 2. In detail, according to an embodiment herein described, said supply line 14 comprises a pump 16 and an opening/ closing valve 17 to allow/ prevent the passage of said organic material M from said pre-treatment tank 13 to said reactor 2. Such an opening/ closing valve 17 can be operated by manual control of the operator or by pre-set digital control deriving from a computer.

Specifically the aforementioned pump 16 arranged along the supply line 14 is a chopping pump for homogenizing and crumbling the biomasses, specifically, vegetable with high density and coarse and uneven grade grain size.

According to the herein described embodiment, said supply line 14 comprises, as well, an auxiliary branch line 18 to reintroduce said organic material M into said pre-treatment tank 13. Such an auxiliary branch line 18 allows to produce an overall stirring of the organic material contained within the pre-treatment tank 13 and, preferably, deposited or sedimented on the bottom, promoting the biological and chemical processes sparked off within the pre-treatment tank 13.

According to the herein described embodiment of the invention, said recirculation means 9 of the apparatus 1 comprise, as well, a first connecting line 20 for transferring part of the organic material M contained in one or more of the reaction chambers 4a, 4b, 5a, 5b, 6a, 6b, 7a, 7b of the reactor 2, or contained in said main line 10, to said pre-treatment tank 13. This allows to introduce, if needed, into the pre-treatment tank 13 some organic material with high bacterial load, since picked up from one of the reaction chambers 12. Of course, this allows to increase the bacterial load of the organic material contained in the pre-treatment tank 13 and, thus promote the pre-treatment processes occurring in the afore mentioned pre-treatment tank 13. It has to be underlined that, due to the presence of the afore mentioned valve members 12, it can be selected the reaction chamber 4a, 4b, 5a, 5b, 6a, 6b, 7a, 7b from which said organic material reaches said pre-treatment tank 13. This is extremely advantageous since, according to the typology of organic material loaded in said pre-treatment tank 13, the more suitable content of organic material to introduce in said pre-treatment tank 13 can be selected, also based on the type of organic material M to be treated, and thus to promote the hydrolysis processes occurring within the tank.

Again, said controlled recirculation means 9 also comprise a second connecting line 21 for transferring part of said organic material contained in one or more of said reaction chambers 4a, 4b, 5a, 5b, 6a, 6b, 7a, 7b, or in said main line 10, to the lagoon 27 for the disposal of the already exhausted material. Note that said second connecting line 21 is present as well as also in said first embodiment of the invention above described.

Advantageously said recirculation means 9 comprise, as well, one evacuation line 22 of organic material, or foams, o other like material, when such an organic material M reaches a certain level, or head, within the last chamber 7b of said reactor 2. Such evacuation line 22 connects, therefore, this latter reaction chamber 7b to said main line 10. This allows to avoid the organic material M, the foam, the crusts or other undesired like material, from exceeding a certain level, or head, within the reactor 2.

Note that, despite the herein described embodiment provides the use of only one evacuation line 22, however an embodiment wherein the number of evacuation lines is provided equal to the number of reaction chambers 4a, 4b, 5a, 5b 6a, 6b, 7a, 7b contained in the reactor 2, still falls within the protection scope of the present invention.

Always according to the herein described, and represented in figure 3, embodiment, said recirculation means 9 comprise a logical unit (herein not shown) for controlling the flow rates of said organic material recirculated between said chambers 4a, 4b, 5a, 5b, 6a, 6b, 7a, 7b, or transferred through said chambers 4a, 4b, 5a, 5b, 6a, 6b, 7a, 7b, or said main line 10, to said pre-treatment tank 13, or said lagoon 27, as a function of the information coming from one or more sensors (herein not shown) of temperature and/ or pH or flow rate of the biogas or density of the organic material M. Such information are obtained through sensors of temperature and/ or pH and/ or flow rate of the biogas and/ or organic material density all placed within the reactor 2.

Finally, according to the herein described embodiment, at one of the upper passages 81, spillway passages, for example, between the reaction chamber 6b and the reaction chamber 7a, a special floating filter 95 is arranged for retaining part of the microbial bacterial flora contained in the organic material M crossing the above mentioned reaction chambers 6b and 7a and being present in dispersed and suspended form in the hydraulic flow rate. This allows to avoid that such a bacterial flora is lost by wash-out and since transferred into lagoon 27 along with the already digested organic material.

According to the embodiments shown in figure 2 and 3, said reactor 2 further comprises, as well, means 150 for heating said reaction chambers 4a, 4b, 5a, 5b, 6a, 6b, 7a, 7b in a controlled way. In practice, in order to aid the digestion processes of the biomass introduced within the reactor 2, the chambers 4a, 4b, 5a, 5b, 6a, 6b, 7a, 7b are conveniently brought to suitably controlled temperatures. In particular, said heating means 150 comprise floor heating members (herein not shown) of the type comprising pipes travelled by a heating fluid, for example.

In accordance with a further embodiment of the invention herein not shown, such heating means 150 are outside of the reactor 2 and comprise heating members of pipe or, alternatively, plate type, for example, placed on the outer wall of the reactor 2 and traveled by a heating liquid. In both the afore mentioned embodiments the heating fluid is heated by a boiler 156 whose operation is known and not further described.

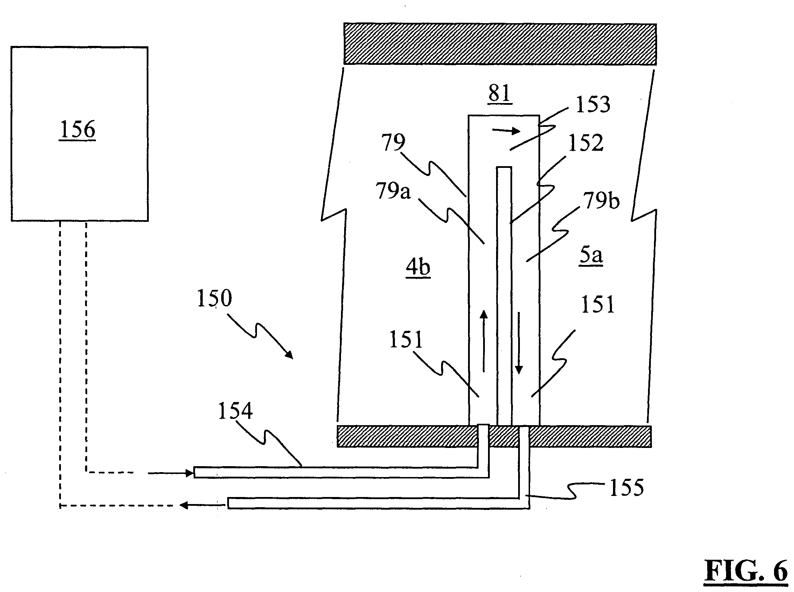

Again, in accordance with a further embodiment of the invention schematically depicted in figure 6, said heating means 150 comprise heating members 151 realized inside the afore mentioned deflectors 79 which separate said reaction chambers 4a, 4b, 5a, 5b, 6a, 6b, 7a, 7b one from another. In a simplified way, the embodiment realized in figure 6 shows only one deflector 79 placed between the two reaction chambers 4b and 5a. The herein depicted deflector 79 is of the type provided with one upper opening 81, or spillway opening. According to the herein described embodiment, the deflector 79 is empty inside and is crossed by said heating liquid. In particular, said deflector 79 is partitioned in two separated regions 79a and 79b partitioned one from each other by a partition 152 and, anyway, fluidically connected through a passage section 153 for the realization of a serpentine path for said heating fluid within said deflector 79. Such heating means 150 further comprise a feed line of the heating fluid 154, which reaches said deflector 79, and at least one return line 155 for said heating fluid, which comes out from said deflector, for the coming out of the already cooled liquid from the deflector. The heating fluid is then sent to a boiler 156, placed outside of the reactor 2, to be heated again. Such a solution allows to uniformly radiate heat within the chambers 4b and 5a of the reactor 2. In addition, differently from the floor heatings, wherein the maintenance of the heating members has to be done periodically due to the fact that the latter are coated shortly with hardened organic material which significantly reduces the radiated thermal power, the heating members 151 realized and contained directly in the deflectors 79 do not need frequent maintenance and allow a significant heat to be developed uniformly, also distributed in height. Note that, despite the embodiment shows heating members 151 realized in the deflectors 79 provided with upper passages 81, however an embodiment wherein said heating members 151 are realized in the deflectors 79 provided with lower passages 80, or else they are realized in some deflectors 79 only and not in all of them, still falls within the protection scope of the present invention.

Incidentally it has to be noted that in a peculiar embodiment, the apparatus for producing biogas 1 by the anaerobic digestion of organic material M, comprises an anaerobic reactor 2 provided with a loading section 3 for said organic material M and a plurality of reaction chambers 4a, 4b, 5a, 5b, 6a, 6b, 7a, 7b arranged in line and connected one to each other by deflectors 79 provided with lower 80 and upper 81 alternate passages, for the forced outflow of said organic material M through each of the said chambers of said plurality of reaction chambers, and means 29 to convey the biogas produced within said reaction chambers outside of said reactor 2. In such an embodiment the reactor 2 is free from controlled recirculation means 9 of said organic material M between the reaction chambers, but it comprises said above described heating means 150.

Again, still according to a further embodiment of the invention, said reactor 2 comprises, as well, means 250 for changing the volume of two chambers 4a and 4b of said reactor 2. In this way the amount of biomass which can be loaded inside one reaction chamber can be increased or decreased, also as a function of the type of organic material to be employed for producing biogas or the requirement of energy to be produced.

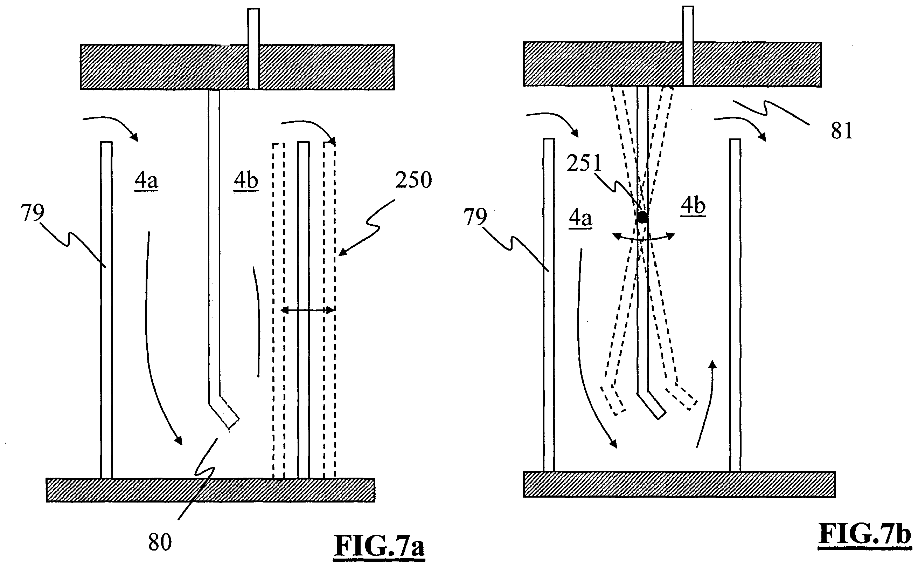

According to an embodiment of the invention, as schematically shown in figure 7a, the deflector 79 partitioning the two reaction chambers 4a and 4b is shiftably constrained to said reactor 2 such to change the volume of the two chambers 4a and 4b.

In a further embodiment of the invention, the deflector 79 partitioning the two reaction chambers 4a and 4b is rotatably constrained to said reactor 2, around the pin 251, so that to change the volume of the said reaction chambers 4a and 4b. In particular, the rotation of the deflector 79 around the pin 251 has two effects. Firstly, it changes the volume of the two chambers which are partitioned by said deflector.

Secondly it changes the geometry of the chambers and, therefore, the path the biomasses followed within the reactor with the consequence that the load losses the biomass is subjected to while crossing the reactor change as well. In fact, once the deflector is rotated, the organic material flow within that specific chamber can be slowed or accelerated, thus increasing or decreasing the biomass passage time in that specific biological compartment.

In addition, according to a specific embodiment of the invention, said means 250 for changing the volume of said reactor 2 comprise a separable wall 252 constrained to the reactor 2 itself. Such a wall 252, preferably the one arranged in opposite position with respect to the position wherein the loading section 3 for said reactor 2 is comprised, can be removed for connecting other two reaction chambers 8a and 8b to the reactor 2 just at said separable wall 252, that is to say a new biological compartment. Note that, despite herein not described, also only one reaction chamber or more than two reaction chambers can be connected to the reactor 2 without thereby departing from the protection scope of the present invention. This has the aim of increasing the volume of the reactor 2 and, therefore, the biogas production of the apparatus.

Note that according to a particular embodiment the reactor 2 is provided with said means for changing the volume 250, but is lacking in said recirculation means 9 for said organic material M.

Finally, said at least one reactor 2 and said pre-treatment tank 13 are realized and sized such to be transportable by road and/ or ship. Furthermore, the sizes of said reactor 2 and said pre-treatment tank 13 correspond to those of the containers transportable by sea or land.

According to the above mentioned embodiments of the invention, said apparatus 1 comprises a system 300 for the electric power generation; such a system, highly schematically represented in figures 2 and 3 since known to the field technician, is connected downstream of the afore mentioned duct 30 for the transportation of the biogas produced by the reactor 2.

Further, according to the invention, a method is provided as well for producing biogas by a production apparatus 1 according to one or more of the claims 1 to 13.

Such a method comprises the step of: a) loading said reactor 2 with said organic material M; and b) forcing the crossing of said plurality of reaction chambers 4a, 4b, 5a, 5b, 6a, 6b, 7a, 7b by said organic material M.

Advantageously, together with the said step b), the method comprises, as well, the step of c) recirculating in a controlled way said organic material M contained within said reactor 2 between said reaction chambers 4a, 4b, 5a, 5b, 6a, 6b, 7a, 7b.

As mentioned above, said recirculation of organic material preferably occurs in a direction opposite to the feed direction of the organic material within the reactor 2.

Again, always according to the method, before said step a) there is the step of d) filling said pre-treatment tank 13 with said organic material M and the step of e) supplying in a controlled way by at least one supply line 14 said one reactor 2 with said organic material M coming from said pre-treatment tank 13.

Again, the method comprises the further step of f) transferring at least part of the organic material M contained in said reaction chambers 4a, 4b, 5a, 5b, 6a, 6b, 7a, 7b, or in said at least one main line 10, to said pre-treatment tank 13, or said lagoon 27.

Finally, said step c) and/ or f) comprises the further step of g) adjusting the flow rate of said organic material M recirculated between said reaction chambers 4a, 4b, 5a, 5b, 6a, 6b, 7a, 7b, or transferred from said reaction chambers 4a, 4b, 5a, 5b, 6a, 6b, 7a, 7b, or said at least one main line, to said at least one pre-treatment tank, or said lagoon, as a function of the information coming from one or more sensors of temperature and/ or pH or flow rate of the biogas or density of the contained organic material.

Finally, always according to the invention, said step b) of the method comprises the further step of g) heating in a controlled way said two chambers 4a, 4b, 5a, 5b, 6a, 6b, 7a, 7b of said plurality of reaction chambers. In particular, according to the herein described embodiment, the temperature in the first two chambers 4a and 4b is slightly higher than that in the additional chambers 5a, 5b 6a, 6b, 7a, 7b. Preferably, the temperature in the first two chambers 4a, 4b is maintained at about 38-39°C whereas in the remaining chambers is maintained at about 36-37°C.

Finally, the method comprises the further step of h) changing the volume of said plurality of reaction chambers 4a, 4b, 5a, 5b, 6a, 6b, 7a, 7b and/ or changing the volume of said reactor 2.

In a particular embodiment, said method for producing biogas by a production apparatus according to one or more of the claims 1 to 13, comprises the step of: a) loading said reactor 2 with said organic material M; and b) forcing the crossing of said plurality of reaction chambers 4a, 4b, 5a, 5b, 6a, 6b, 7a, 7b by said organic material M. In addition the method comprises the further step of h) changing the volume of said plurality of reaction chambers 4a, 4b, 5a, 5b, 6a, 6b, 7a, 7b and/ or said reactor 2.

## Claims

1. Apparatus (1) for producing biogas by the anaerobic digestion of organic material (M), comprising at least one anaerobic reactor (2) provided with a loading section (3) for said organic material and a plurality of reaction chambers (4a, 4b, 5a, 5b, 6a, 6b, 7a, 7b), arranged in line and connected one to each other by deflectors (79) provided with lower (80) and upper (81) alternate passages, for the outflow of said organic material through each of said chambers of said plurality of reaction chambers, and means (29) to convey the biogas produced within said one or more reaction chambers outside of said at least one reactor, said at least one reactor comprising, as well, means (9) for the recirculation of said organic material between one or more chambers of said plurality of reaction chambers, wherein said recirculation means (9) comprise, outside of said at least one reactor, at least one main line (10) fluidically connecting one or more reaction chambers one to each other, at least one or more inflow/outflow lines (90) for said organic material connected, at a first end (90a), to said at least one main line and, at least a second end (90b), to at least one chamber of said one or more reaction chambers, at least one recirculation pump (11) for said organic material operating within said at least one main line and/or said one or more inflow/outflow lines, and valve means (12) for connecting by control said one or more reaction chambers to said at least one main line of fluidic connection, said apparatus further comprising at least one pre-treatment tank (13) of said organic material and at least one line (14) for the controlled supply through said at least one loading section (3) of said at least one reactor with said organic material deriving from said at least one pre-treatment tank, **characterized in that** said means of controlled recirculation comprise as well at least one first connecting line (20) for transferring at least part of the organic material contained in said at least one main line to said at least one pretreatment tank.

2. Apparatus according to claim 1, **characterized in that** said at least one second end of said one or more outflow/ inflow lines is arranged beneath said one or more chamber of said plurality of chambers, for the pickup of the solid component of said organic material, and/ or at the level of the head of said organic material present in said one or more chambers, for the pickup of the liquid component of said organic material, or the foam, or the surface crust of said organic material.

3. Apparatus according to one or claims from 1 to 2, **characterized in that** said at least one supply line (14) comprises at least one pump (16) and at least one opening/ closing valve (17) to allow/ prevent the passage of said organic material from said at least one pre-treatment tank to said at least one reactor.

4. Apparatus according to claim 3, **characterized in that** said supply line comprises, as well, at least one auxiliary branch line (17) for reintroducing said organic material into said pre-treatment tank.

5. Apparatus according to one or more of the claims 1 to 4, **characterized in that** said means of controlled recirculation comprise as well at least a second connecting line (21) for transferring at least part of the organic material contained in one or more of said reaction chambers, or in said at least one main line, to the lagoon (27) for the disposal of the exhausted material, or to the storage or the like.

6. Apparatus according to one or more of the preceding claims, **characterized in that** said recirculation means comprise, as well, one or more evacuation lines (22) of the organic material, or foams, or other like material, when a certain level within one or more chambers of said plurality of chambers is reached, each of said one or more evacuation lines connecting at least one reaction chamber to said at least one main line.

7. Apparatus according to one or more of the preceding claims, **characterized in that** said recirculation means comprise at least one unit for controlling the flow rates of said organic material recirculated between said one or more chambers, or transferred from said one or more reaction chambers, or said at least one main line, to said pre-treatment tank or said lagoon, as a function of the information coming from one or more sensors of temperature and/ or pH and/ or flow rate biogas or of density of organic material.

8. Apparatus according to one or more of the preceding claims, **characterized in that** said at least one reactor comprises as well means for heating in a controlled way said one or more chambers of said plurality of reaction chambers.

9. Apparatus according to one or more of the preceding claims, **characterized in that** said at least one reactor comprises means to break the crust formed on the surface of said organic material.

10. Apparatus according to one or more of the preceding claims, **characterized in that** said at least one reactor (2) comprises as well means for changing the volume of one or more chambers of said at least one reactor and/ or the volume of said at least one reactor.

11. Method for producing biogas by a production apparatus according to one or more of the claims 1 to 10, comprising the step of:
a) loading said at least one reactor (2) with said organic material;
b) carrying out the crossing of plurality of reaction chambers by said organic material;
c) recirculating in a controlled way said organic material contained in said at least one reactor between said one or more chambers of said plurality of reaction chambers,
wherein, before said step a) the step is comprised of d) filling said at least one pre-treatment tank (13) with said organic material and the step of e) supplying in a controlled way by at least one supply line (14) said at least one reactor with said organic material coming from said at least one pre-treatment tank, said method being **characterized by** comprising the further step of f) transferring at least part of the organic material contained in said at least one main line to said at least one pre-treatment tank.

12. Method according to claim 11, **characterized in that** said step c) and/or f) comprises the further step of g) adjusting the flow rate of said organic material recirculated between said one or more reaction chambers of said plurality of reaction chambers, or transferred from said one or more reaction chambers, or said at least one main line, to said at least one pre-treatment tank, or said lagoon, or the storage, as a function of the information coming from one or more sensors of biogas temperature and/ or pH or flow rate or of density of contained organic material.

13. Method according to one or more of the claims from 11 to 12, **characterized in that** said step b) comprises the further step of g) heating in a controlled way said one or more chambers of said plurality of reaction chambers.

## Patentansprüche

1. Vorrichtung (1) zur Erzeugung von Biogas durch de anaeroben Aufschluss von organischem Material (M), umfassend mindestens einen anaeroben Reaktor (2), bereitgestellt mit einem Beladungsabschnitt (3) für das organische Material und einer Vielzahl von Reaktionskammern (4a, 4b, 5a, 5b, 6a, 6b, 7a, 7b), angeordnet in einer Linie und durch Defektoren (79) miteinander verbunden sind, die mit unteren (80) und oberen (81) alternierenden Durchgängen versehen sind, für den Abfluss des organischen Materials durch jede der Kammern der Vielzahl von Reaktionskammern, und Mittel (29), um das in der einen oder den mehreren Reaktionskammern erzeugte Biogas außerhalb des mindestens einen Reaktors zu befördern, wobei der mindestens eine Reaktor auch Mittel (9) zur Rezirkulierung des organischen Materials zwischen einer oder mehreren Kammern der Vielzahl von Reaktionskammern umfasst, wobei die Rezirkulationsmittel (9) außerhalb des mindestens einen Reaktors mindestens eine Hauptleitung (10) umfassen, die eine oder mehrere Reaktionskammern miteinander fluidisch verbinden, mindestens eine oder mehrere Zufluss-/Abflussleitungen (90) für das organische Material, die an einem ersten Ende (90a) mit der mindestens einen Hauptleitung und an mindestens einem zweiten Ende (90b) mit mindestens einer Kammer der einen oder mehreren Reaktionskammern verbunden sind, mindestens eine Rezirkulationspumpe (11) für das organische Material, die innerhalb der mindestens einen Hauptleitung und/oder der einen oder der mehreren Zufluss-/Abflussleitungen arbeitet, und eine Ventileinrichtung (12) zum gesteuerten Verbinden der einen oder der mehreren Reaktionskammern mit der mindestens einen Hauptleitung der fluidischen Verbindung, wobei die Vorrichtung mindestens einen Vorbehandlungstank (13) des organischen Materials und mindestens eine Leitung (14) für die gesteuerte Versorgung durch den mindestens einen Beladungsabschnitt (3) des mindestens einen Reaktors mit dem organischen Material, das aus dem mindestens einen Vorbehandlungstank abgeleitet ist, umfasst, **dadurch gekennzeichnet, dass** das Mittel zum gesteuerten Rezirkulieren auch mindestens eine Verbindungsleitung (20) zum Transferieren mindestens eines Teils des organischen Materials, das in der mindestens einen Hauptleitung zu dem mindestens einen Vorbehandlungstank enthalten ist, umfasst.

2. Vorrichtung nach Anspruch 1, **dadurch gekennzeichnet, dass** das mindestens eine zweite Ende der einen oder mehreren Abfluss-/Zuflussleitungen unter der einen oder den mehreren Kammern der Vielzahl von Kammern angeordnet ist, um die feste Komponente des organischen Materials aufzunehmen; und/oder auf der Höhe des Kopfes des in der einen oder den mehreren Kammern vorhandenen organischen Materials zur Aufnahme der flüssigen Komponente des organischen Materials oder des Schaums oder der Oberflächenkruste des organischen Materials.

3. Vorrichtung nach einem der Ansprüche 1 oder 2, **dadurch gekennzeichnet, dass** die mindestens eine Versorgungsleitung (14) mindestens eine Pumpe (16) und mindestens ein Öffnungs-/Schließventil (17) umfasst, um den Durchgang des organischen Materials von dem mindestens einen Vorbehandlungstank zu dem mindestens einen Reaktor zu ermöglichen/zu verhindern.

4. Vorrichtung nach Anspruch 3, **dadurch gekennzeichnet, dass** die Versorgungsleitung auch mindestens eine Hilfszweigleitung (17) zum Wiedereinführen des organischen Materials in den Vorbehandlungstank umfasst.

5. Vorrichtung nach einem oder mehreren der Ansprüche 1 bis 4, die **dadurch gekennzeichnet sind, dass** das Mittel zur gesteuerten Rezirkulierung auch mindestens eine zweite Verbindungsleitung (21) zum Transferieren mindestens eines Teils des in einer oder mehreren der Reaktionskammern enthaltenen organischen Materials oder in mindestens einer Hauptleitung zum Teich (27) zur Entsorgung des verbrauchten Materials oder zur Lagerung oder dergleichen umfasst.

6. Vorrichtung nach einem oder mehreren der vorhergehenden Ansprüche, die **dadurch gekennzeichnet ist, dass** die Rezirkulationsmittel auch eine oder mehrere Evakuierungsleitungen (22) des organischen Materials oder Schaums oder eines ähnlichen Materials, wenn ein bestimmtes Niveau innerhalb eines oder mehrerer Kammern der Vielzahl von Kammern erreicht ist, umfassen, wobei jede der einen oder der mehreren Evakuierungsleitungen mindestens eine Reaktionskammer mit der mindestens eine Hauptleitung verbindet.

7. Vorrichtung nach einem oder mehreren der vorhergehenden Ansprüche, **dadurch gekennzeichnet, dass** die Rezirkulationsmittel mindestens eine Einheit zum Steuern der Strömungsraten des organischen Materials umfassen, das zwischen der einen oder den mehreren Kammern rezirkuliert oder von der einen oder den mehreren Reaktionskammern oder der mindestens einen Hauptleitung zu dem Vorbehandlungstank oder dem Teich transferiert wird als eine Funktion der Information, die von einem oder mehreren Sensoren für Temperatur und/oder pH-Wert und/oder Durchflussrate des Biogases oder für die Dichte des organischen Materials kommt.

8. Vorrichtung nach einem oder mehreren der vorhergehenden Ansprüche, **dadurch gekennzeichnet, dass** der mindestens eine Reaktor auch Mittel zum gesteuerten Erwärmen der einen oder mehreren Kammern der Vielzahl von Reaktionskammern umfasst.

9. Vorrichtung nach einem oder mehreren der vorhergehenden Ansprüche, **dadurch gekennzeichnet, dass** der mindestens eine Reaktor Mittel zum Brechen der auf der Oberfläche des organischen Materials gebildeten Kruste umfasst.

10. Vorrichtung nach einem oder mehreren der vorhergehenden Ansprüche, **dadurch gekennzeichnet, dass** der mindestens eine Reaktor (2) auch Mittel zum Ändern des Volumens der einen oder mehreren Kammern des mindestens einen Reaktors und/oder des Volumens des mindestens einen Reaktors umfasst.

11. Verfahren zur Erzeugung von Biogas durch eine Produktionsvorrichtung nach einem oder mehreren der Ansprüche 1 bis 10, umfassend den Schritt:
a) Beladen des mindestens einen Reaktors (2) mit dem organischen Material;
b) Durchführen der Durchkreuzung der Vielzahl von Reaktionskammern durch das organische Material;
c) gesteuertes Rezirkulieren des in dem mindestens einen Reaktor enthaltenen organischen Materials zwischen der einen oder den mehreren Kammern der Vielzahl von Reaktionskammern,
wobei vor dem Schritt a) der Schritt umfasst d) Füllen des mindestens einen Vorbehandlungstanks (13) mit dem organischen Material und den Schritt e) gesteuertes Versorgen durch mindestens eine Versorgungsleitung (14) mit dem organischem Material des mindestens einen Reaktors aus dem mindestens einen Vorbehandlungstank, wobei das Verfahren **dadurch gekennzeichnet ist, dass** es den zusätzlichen Schritt f) Transferieren mindestens einen Teils des organischen Materials, welches in der mindestens einen Hauptleitung enthalten ist, an den mindestens einen Vorbehandlungstank.

12. Verfahren nach Anspruch 11, **gekennzeichnet dadurch, dass** der Schritt c) und/oder f) den weiteren Schritt g) Einstellen der Fließgeschwindigkeit des organischen Materials umfasst, das zwischen der einen oder den mehreren Reaktionskammern der Vielzahl von Reaktionskammern zirkuliert oder von einem oder mehreren Reaktionskammern von der mindestens einen Hauptleitung zu dem mindestens einen Vorbehandlungstank oder dem Teich oder dem Speicher transferiert wird als eine Funktion der Information, die von einem oder mehreren Sensoren der Biogastemperatur und/oder des pH-Werts oder der Durchflussrate oder der Dichte des enthaltenen organischen Materials kommt.

13. Verfahren nach einem oder mehreren der Ansprüche 11 bis 12, **dadurch gekennzeichnet, dass** der Schritt b) den weiteren Schritt g) gesteuertes Erwärmen der einen oder mehreren Kammern der Vielzahl von Reaktionskammern umfasst.

## Revendications

1. Appareil (1) destiné à produire du biogaz par la digestion anaérobie de matière organique (M), comprenant au moins un réacteur anaérobie (2) doté d'une section de chargement (3) pour ladite matière organique et d'une pluralité de chambres de réaction (4a, 4b, 5a, 5b, 6a, 6b, 7a, 7b), disposées en ligne et raccordées les unes aux autres par des déflecteurs (79) dotés de passages inférieurs (80) et supérieurs (81) alternés, pour la sortie de ladite matière organique à travers chacune desdites chambres de ladite pluralité de chambres de réaction, et d'un moyen (29) pour transporter le biogaz produit à l'intérieur desdites une ou plusieurs chambres de réaction à l'extérieur dudit au moins un réacteur, ledit au moins un réacteur comprenant, aussi, un moyen (9) pour la recirculation de ladite matière organique entre une ou plusieurs chambres de ladite pluralité de chambres de réaction, dans lequel ledit moyen de recirculation (9) comprend, à l'extérieur dudit au moins un réacteur, au moins une voie principale (10) reliant de manière fluidique une ou plusieurs chambres de réaction les unes aux autres, au moins une ou plusieurs voies d'entrée/de sortie (90) pour ladite matière organique raccordées, au niveau d'une première extrémité (90a), à ladite au moins une voie principale et, au moins une seconde extrémité (90b), à au moins une chambre desdites une ou plusieurs chambres de réaction, au moins une pompe de recirculation (11) pour ladite matière organique fonctionnant à l'intérieur de ladite au moins une voie principale et/ou desdites une ou plusieurs voies d'entrée/de sortie, et un moyen de soupape (12) pour raccorder par commande lesdites une ou plusieurs chambres de réaction à ladite au moins une voie principale de liaison fluidique, ledit appareil comprenant en outre au moins un réservoir de prétraitement (13) de ladite matière organique et au moins une voie (14) pour l'alimentation régulée à travers ladite au moins une section de chargement (3) dudit au moins un réacteur avec ladite matière organique provenant dudit au moins un réservoir de prétraitement, **caractérisé en ce que** ledit moyen de recirculation commandée comprend aussi au moins une première voie de raccordement (20) pour transférer au moins une partie de la matière organique contenue dans ladite au moins une voie principale vers ledit au moins un réservoir de prétraitement.

2. Appareil selon la revendication 1, **caractérisé en ce que** ladite au moins une seconde extrémité desdites une ou plusieurs voies d'entrée/de sortie est disposée en dessous desdites une ou plusieurs chambres de ladite pluralité de chambres, pour la récupération du composant solide de ladite matière organique, et/ou au niveau de la tête de ladite matière organique présente dans lesdites une ou plusieurs chambres, pour la récupération du composant liquide de ladite matière organique, ou de la mousse, ou de la croûte superficielle de ladite matière organique.

3. Appareil selon l'une des revendications 1 à 2, **caractérisé en ce que** ladite au moins une voie d'alimentation (14) comprend au moins une pompe (16) et au moins une soupape d'ouverture/de fermeture (17) pour permettre/prévenir le passage de ladite matière organique dudit au moins un réservoir de prétraitement vers ledit au moins un réacteur.

4. Appareil selon la revendication 3, **caractérisé en ce que** ladite voie d'alimentation comprend, aussi, au moins une voie de branchement auxiliaire (17) pour réintroduire ladite matière organique dans ledit réservoir de prétraitement.

5. Appareil selon l'une ou plusieurs des revendications 1 à 4, **caractérisé en ce que** ledit moyen de recirculation commandée comprend aussi au moins une seconde voie de raccordement (21) pour transférer au moins une partie de la matière organique contenue dans une ou plusieurs desdites chambres de réaction, ou dans ladite au moins une voie principale, vers le bassin (27) pour l'élimination de la matière évacuée, ou pour son stockage ou analogue.

6. Appareil selon l'une ou plusieurs des revendications précédentes, **caractérisé en ce que** ledit moyen de recirculation comprend, aussi, une ou plusieurs voies d'évacuation (22) de la matière organique, ou de mousses, ou d'une autre matière similaire, lorsqu'un certain niveau à l'intérieur d'une ou plusieurs chambres de ladite pluralité de chambres est atteint, chacune desdites une ou plusieurs voies d'évacuation raccordant au moins une chambre de réaction à ladite au moins une voie principale.

7. Appareil selon l'une ou plusieurs des revendications précédentes, **caractérisé en ce que** ledit moyen de recirculation comprend au moins une unité de régulation des débits de ladite matière organique remise en circulation entre lesdites une ou plusieurs chambres, ou transférée desdites une ou plusieurs chambres de réaction, ou de ladite au moins une voie principale, vers ledit réservoir de prétraitement ou ledit bassin, en fonction des informations provenant d'un ou plusieurs capteurs de température et/ou de pH et/ou de débit de biogaz ou de la densité de la matière organique.

8. Appareil selon l'une ou plusieurs des revendications précédentes, **caractérisé en ce que** ledit au moins un réacteur comprend aussi un moyen de chauffage d'une manière régulée desdites une ou plusieurs chambres de ladite pluralité de chambres de réaction.

9. Appareil selon l'une ou plusieurs des revendications précédentes, **caractérisé en ce que** ledit au moins un réacteur comprend un moyen pour casser la croûte formée à la surface de ladite matière organique.

10. Appareil selon l'une ou plusieurs des revendications précédentes, **caractérisé en ce que** ledit au moins un réacteur (2) comprend aussi un moyen de variation du volume de l'une ou plusieurs chambres dudit au moins un réacteur et/ou du volume dudit au moins un réacteur.

11. Procédé de production de biogaz par un appareil de production selon l'une ou plusieurs des revendications 1 à 10, comprenant les étapes consistant à :
a) charger ledit au moins un réacteur (2) de ladite matière organique ;
b) réaliser le passage à travers la pluralité de chambres de réaction de ladite matière organique ;
c) remettre en circulation d'une manière régulée ladite matière organique contenue dans ledit au moins un réacteur entre lesdites une ou plusieurs chambres de ladite pluralité de chambres de réaction,
dans lequel, avant ladite étape a) l'étape est composée de d) remplir ledit au moins un réservoir de prétraitement (13) avec ladite matière organique et l'étape e) alimenter d'une manière régulée par au moins une voie d'alimentation (14) ledit au moins un réacteur avec ladite matière organique provenant dudit au moins un réservoir de prétraitement, ledit procédé étant **caractérisé par** le fait de comprendre l'étape supplémentaire consistant à f) transférer au moins une partie de la matière organique contenue dans ladite au moins une voie principale vers ledit au moins un réservoir de prétraitement.

12. Procédé selon la revendication 11, **caractérisé en ce que** ladite étape c) et/ou f) comprend l'étape supplémentaire consistant à g) ajuster le débit de ladite matière organique remise en circulation entre lesdites une ou plusieurs chambres de réaction de ladite pluralité de chambres de réaction, ou transférée desdites une ou plusieurs chambres de réaction, ou de ladite au moins une voie principale, vers au moins un réservoir de prétraitement, ou ledit bassin, ou le stockage, en fonction des informations provenant d'un ou plusieurs capteurs de la température et/ou du pH ou du débit du biogaz ou de la densité de la matière organique contenue.

13. Procédé selon l'une ou plusieurs des revendications 11 à 12, **caractérisé en ce que** ladite étape b) comprend l'étape supplémentaire consistant à g) chauffer d'une manière régulée lesdites une ou plusieurs chambres de ladite pluralité de chambres de réaction.
